# EUROPÄISCHE PATENTSCHRIFT

(11) **EP 0 862 897 B1**
(45) Veröffentlichungstag und Bekanntmachung des Hinweises auf die Patenterteilung: **11.08.2004**
(21) Anmeldenummer: 98103930.8
(22) Anmeldetag: 05.03.1998
(51) Int. Cl.: A61B 5/00, A61C 19/04, G01N 21/64

(54) **Fluoreszenz-Messverfahren und Vorrichtung zum Ermitteln von Karies an Zähnen**
Fluorescence measurement method and device for detecting dental caries
Procédé pour mesurer la fluorescence et dispositif pour détecter des caries dentaires

(30) Priorität: 07.03.1997 DE 19709500
(43) Veröffentlichungstag der Anmeldung: 09.09.1998
(73) Patentinhaber: Kaltenbach & Voigt GmbH & Co. KG, 88400 Biberach/Riss (DE)
(72) Erfinder: Lussi, Dr.Adrian, 3076-Worb (CH); Eibofner, Eugen, 88400 Biberach (DE); Klotz, Markus, 75378 Bad Liebenzell (DE); Bareiss, Manfred, 73642 Welzheim (DE)
(74) Vertreter: Schmidt-Evers, Jürgen, Dipl.-Ing.

(56) Entgegenhaltungen:
- DE-A- 3 345 465
- US-A- 5 382 163

## Beschreibung

Die vorliegende Erfindung betrifft eine Vorrichtung zur Ermittlung von Karies, Plaque oder bakteriellen Befall an Zähnen. Ferner betrifft die Erfindung ein Verfahren zum Lokalisieren einer Stelle mit erhöhter Fluoreszenzintensität an einem zu untersuchenden Objekt, insbesondere an einem Zahn.

Bekannterweise kann Karies an Zähnen durch visuelle Untersuchung oder durch Verwendung von Röntgenstrahlen entdeckt werden. Mit Hilfe einer visuellen Untersuchung lassen sich jedoch häufig keine zufriedenstellende Ergebnisse erzielen, da sich beispielsweise Karies im Frühstadium oder an einem schwer einsehbaren Zahnbereich nicht feststellen läßt. Aufgrund der schädigenden Wirkung von Röntgenstrahlen für die menschliche Gesundheit ist auch die Untersuchung mit Hilfe von Röntgenstrahlen nicht optimal.

Es wurde daher ein berührungsloses Untersuchungsverfahren zum Feststellen von Karies an Zähnen vorgeschlagen, wobei der Zahn mit einer nahezu monochromatischen Lichtquelle bestrahlt wird. Aufgrund der Bestrahlung des Zahns mit monochromatischem Licht wird an dem Zahn eine Fluoreszenzstrahlung angeregt, wobei das Fluoreszenzspektrum deutliche Unterschiede zwischen kariösen und gesunden Zahnbereichen aufweist. Aufgrund des erfaßten Fluoreszenzspektrums eines zu untersuchenden Zahns kann somit eindeutig ein gesunder Zahnbereich von einem kariösen Zahnbereich unterschieden werden. Entsprechende zahnärztliche Vorrichtungen zum Erkennen von Karies sind beispielsweise aus der DE 30 31 249 C2, DE 42 00 741 A1 und DE 93 17 984 U1 bekannt. Ferner beschreibt auch die US 5,382,163 eine Verfahren zur berührungslosen Kariesdiagnose.

Aus der DE 33 45 465 A1 schließlich ist eine Diagnosegerät bekannt, welches ebenfalls auf einem optischen Verfahren beruht. Wiederum wird ein zu untersuchender Zahn mit mehrere Anregungsstrahlungs-Pulsen bestrahlt, wobei die Anzahl der abgegebenen Pulse in einem ersten Speicherelement registriert wird. Für jeden einzelnen Anregungspuls wird überprüft, ob die von dem Zahn zurückgeworfene Strahlung auf Karies hindeutet. Die Anzahl dieser positiven Ereignisse wird dann in einem zweiten Speicherelement registriert, wobei letztendlich anhand des Verhältnisses des beiden Speicherwerte bestimmt wird, ob der Zahn kariös ist oder nicht. Mit Hilfe dieser bekannt Vorrichtungen läßt sich allerdings lediglich erkennen, ob ein untersuchter Zahn insgesamt kariös ist oder nicht. Im Falle eines kariösen Zahns wird bei den bekannten Vorrichtungen beispielsweise eine entsprechende optische oder akustische Meldung ausgegeben. An einem erkannten kariösen Zahn kann jedoch die Stärke des Kariesbefalls schwanken. Nach Beendigung der Untersuchung des Zahns ist es dann wünschenswert, diejenige Stelle des Zahns wiederzufinden, die den stärksten Kariesbefall aufweist, um die entsprechende Zahnstelle behandeln zu können. Dies ist jedoch mit den bekannten Vorrichtungen nicht möglich.

Der vorliegenden Erfindung liegt daher die Aufgabe zugrunde, eine Vorrichtung zum Erkennen von Karies an Zähnen vorzuschlagen, mit deren Hilfe die Zahnstelle mit dem stärksten Kariesbefall wieder aufgefunden werden kann, nachdem ein Zahn als kariös erkannt worden ist. Ferner liegt der Erfindung die Aufgabe zugrunde, ein Verfahren anzugeben, mit dem diejenige Stelle auf einfache Weise lokalisiert werden kann, welche die höchste Fluoreszenzintensität aufweist und dementsprechend aller Voraussicht nach von Karies befallen ist.

Diese Aufgaben werden durch ein Verfahren gemäß Anspruch 1 und eine Vorrichtung gemäß Anspruch 6 gelöst.

Die Unteransprüche beschreiben weitere vorteilhafte Ausgestaltungen der Erfindung.

Erfindungsgemäß wird während der Untersuchung eines Zahnes auf Kariesbefall der jeweils ermittelte Spitzenwert der an dem Zahn angeregten Fluoreszenzstrahlung ermittelt und gespeichert. Nach der Erkennung, daß es sich bei dem untersuchten Zahn um einen kariösen Zahn handelt, kann die Stelle mit dem stärksten Kariesbefall durch erneutes Abtasten des Zahns mit der Anregungsstrahlung aufgefunden werden, in dem der sich durch die erneute Bestrahlung ergebende Meßwert jeweils mit dem Spitzenwert verglichen wird. Stimmt der momentane Meßwert mit dem gespeicherten Spitzenwert überein, so handelt es sich bei der diesem Meßwert entsprechenden Zahnstelle um die Stelle mit dem stärksten Kariesbefall. Das Auffinden der Zahnstelle mit dem stärksten Kariesbefall kann dadurch erleichtert werden, daß während des erneuten Abtastens ein akustisches Signal erzeugt wird, dessen Frequenz mit steigendem Meßwert ansteigt, wobei insbesondere das akustische Signal nur solange ausgegeben wird, solange der Meßwert ansteigt. Alternativ kann auch ein akustisches Signal nur dann ausgegeben werden, wenn der Momentanwert der erneuten Untersuchung des Zahns mit dem gespeicherten Spitzenwert übereinstimmt.

Es ist zu beachten, daß der angezeigte Spitzenwert nicht zwangsläufig dem Maximalwert der angeregten Fluoreszenzstrahlung entsprechen muß, sondern auch auf andere Weise ermittelt und aus der Fluoreszenzstrahlung abgeleitet werden kann.

Jeder Zahn besitzt eine unterschiedliche eigenen Fluoreszenz. Da diese eigene Fluoreszenz der Messung stören kann, wird erfindungsgemäß vorgeschlagen, vor Beginn der Messung einen Abgleichvorgang durchzuführen, wobei dieser Abgleich durch Messen eines gesunden Zahnbereiches durchgeführt wird. Durch den Abgleich wird der Zähler bzw. Speicher für den Momentanmeßwert auf Null gesetzt.

Erfindungsgemäß sollen zur Untersuchung eines Zahns unterschiedliche Arten von Lichtsonden einsetzbar sein, wobei die Lichtsonden auf ein zahnärztliches Handstück abnehmbar aufgesteckt werden. Nach Aufstecken einer neuen Lichtsonde auf das zahnärztliche Handstück muß diese erfindungsgemäß zunächst kalibriert werden, wobei zu diesem Zweck die Lichtsonde mit ihrer Spitze senkrecht auf ein Referenzwerkstück, z.B. ein Keramikstück, aufgesetzt und vermessen wird.

Ein Ausführungsbeispiel der Erfindung wird nachfolgend unter Bezugnahme auf die Zeichnung näher beschrieben. Es zeigen:
Figur 1 zeigt eine Gesamtansicht der erfindungsgemäßen Vorrichtung zum Erkennen von Karies,
Figur 2 zeigt ein vereinfachtes Blockschaltbild des internen Aufbaus der in Figur 1 gezeigten Vorrichtung,
Figur 3 zeigt eine Querschnittsansicht des bei der erfindungsgemäßen Vorrichtung verwendeten zahnärztlichen Handstücks,
Figur 4a zeigt den Vorgang der Kalibrierung einer Lichtsonde, und
Figur 4b zeigt den Vorgang eines Abgleichs der erfindungsgemäßen Vorrichtung zum Erkennen von Karies.

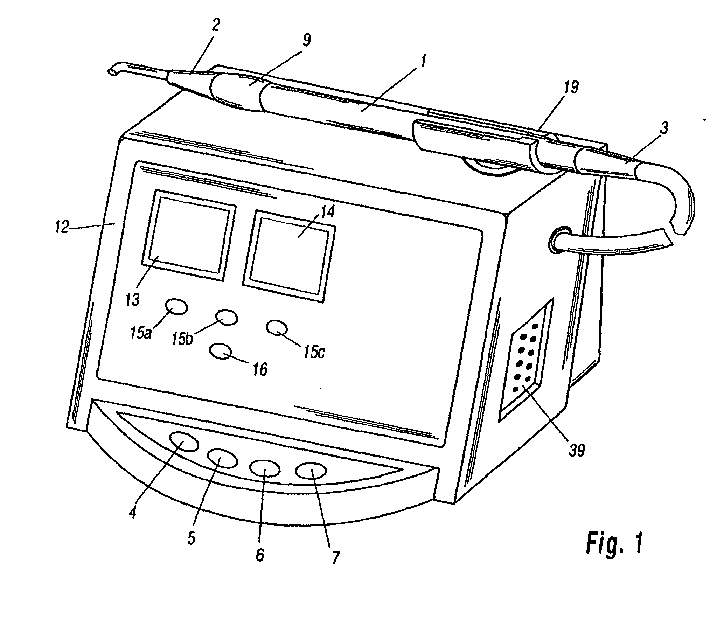

Figur 1 zeigt eine Gesamtansicht der erfindungsgemäßen Vorrichtung zum Erkennen von Karies, Plaque oder bakteriellem Befall an Zähnen. Die erfindungsgemäße Vorrichtung kann jedoch allgemein zum Feststellen eines kranken Gewebes eingesetzt werden, dessen Fluoreszenzstrahlung sich von derjenigen eines gesunden Gewebes unterscheidet. Dabei muß es sich nicht um ein Zahngewebe, sondern allgemein um ein menschliches oder tierisches Körpergewebe handeln. Die in Figur 1 gezeigt Vorrichtung umfaßt ein Gehäuse 12, welches über einen Versorgungsschlauch 3 mit einem zahnärztlichen Handstück 1 und einer auf das zahnärztliche Handstück aufgesteckten Lichtsonde 2 verbunden ist. Um das Gewicht der erfindungsgemäßen Vorrichtung zu reduzieren und die Mobilität zu erhöhen, wird vorteilhafterweise die Vorrichtung mit einer Batterie oder einem Akkumulator als Spannungsquelle versorgt. Aus diesem Grund weist die Frontseite des Gehäuses 12 eine Leuchtdiode 16 auf, bei deren Aufleuchten der Benutzer darauf hingewiesen wird, daß die Versorgungsspannung unter einen bestimmten Grenzwert abgesunken ist, wodurch der Benutzer zum Auswechseln der Batterie bzw. Aufladen des Akkumulators aufgefordert wird. Des weiteren weist die erfindungsgemäße Vorrichtung drei Anzeigen 15a - 15c auf, die die auf das zahnärztliche Handstück 1 aufgesteckte Lichtsonde 2 bezeichnen. Im vorliegenden Fall kann zwischen drei unterschiedlichen Lichtsonden, abhängig von der Art des zu untersuchenden Zahnbereiches, ausgewählt werden. Die Anzeigen 13 und 14 dienen zur Anzeige des augenblicklichen Fluoreszenzspektrum-Meßwerts bzw. des Spitzenwerts. Wie noch nachfolgend näher beschrieben wird, dienen die Tasten 4 - 7 unter anderem zur Auswahl vorgegebener Betriebsmodi und zur Auswahl der auf das zahnärztliche Handstück 1 aufgesteckten Lichtsonde 2. Das zahnärztliche Handstück 1 weist einen Ringschalter 9 auf, wobei der Ringschalter 9 eine sich in Umfangrichtung des zahnärztlichen Handstücks 1 ringförmig erstreckende Schaltfläche aufweist, so daß der Ringschalter 9 einfach durch Fingerdruck unabhängig von der Lage des zahnärztlichen Handstücks 1 in der Hand der Bedienperson betätigt werden kann. Der Ringschalter 8 dient insbesondere zum Ein- / Ausschalten der Vorrichtung sowie zum Löschen des Spitzenwerts und Abgleichen der Lichtsonde 2. Zur Verbesserung der Transportabilität der erfindungsgemäßen Vorrichtung ist eine Halterung 19 vorgesehen, in die das zahnärztliche Handstück 1 mit der Lichtsonde 2 abgelegt werden kann. Vorteilhafterweise weist das Gehäuse 12 auch eine (nicht gezeigte) seitliche Ausnehmung auf, in die die Halterung alternativ eingesteckt werden kann, um das zahnärztliche Handstück 1 auch seitlich an dem Gehäuse 12 befestigen und ablegen zu können. Die Halterung 19 ist gegenüber dem Gehäuse 12 drehbar, wobei die Drehung entweder stufenlos oder in Übereinstimmung mit vorgegebenen Raststufen erfolgen kann. Die Vorrichtung kann schließlich auch Befestigungsmittel (z.B. am Boden des Gehäuses) aufweisen, um sie an einer geeigneten Stelle an einem zahnärztlichen Arbeitsplatz befestigen zu können. Die Befestigung kann beispielsweise mit Hilfe eines Winkels erfolgen, der mit Aufnahmezapfen an der Vorrichtung (beispielsweise in einer Aufnahmeöffnung am Boden der Vorrichtung) und mit einem Klebestreifen an einer geeigneten Stelle eines zahnärztlichen Arbeitsplatzes befestigt wird. Durch eine geeignete Gestaltung des Winkels kann die Vorrichtung in horizontaler und vertikaler Richtung gedreht oder gekippt und somit optimal an die Blickrichtung eines Benutzers angepaßt werden. Des weiteren sind Schraubbefestigungen vorstellbar.

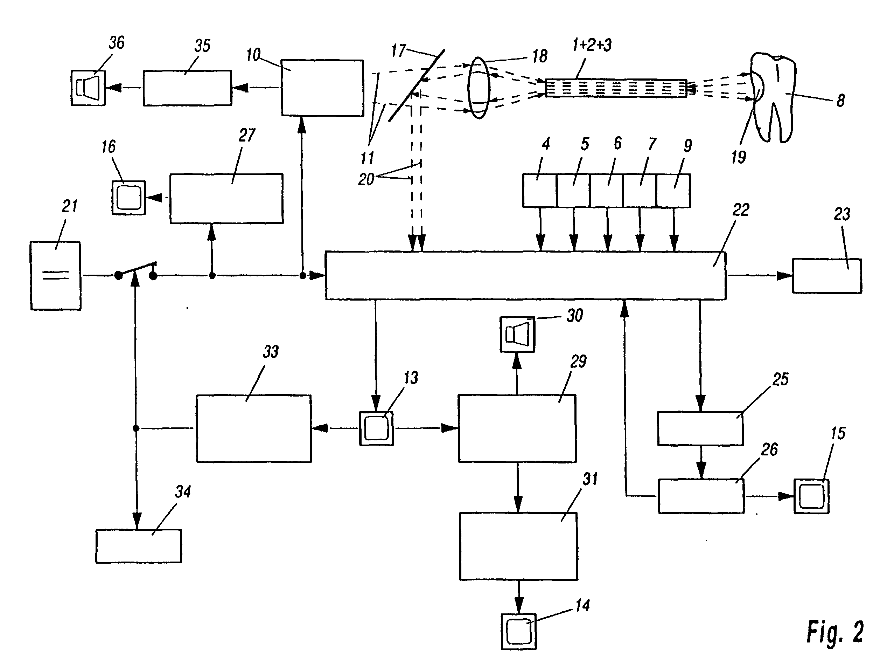

Die Funktion der in Figur 1 gezeigten erfindungsgemäßen Vorrichtung wird nachfolgend unter Bezugnahme auf Figur 2 näher erläutert.

Wie bereits erwähnt, handelt es sich bei der erfindungsgemäßen Vorrichtung um eine Vorrichtung, die Karies an Zähnen mit Hilfe der Bestrahlung eines zu untersuchenden Zahns 8 ermittelt. Die das Anregungslicht erzeugende Lichtquelle 10 kann beispielsweise ein HeNe-Laser oder eine Laserdiode sein, die vorteilhafterweise die Anregungsstrahlung 11 mit einer Wellenlänge im Bereich 600 nm bis 670 nm erzeugt. Insbesondere ist eine Anregungswellenlänge von ca. 655 nm vorteilhaft. Die Anregungsstrahlung 11 wird über ein Linsensystem 18 oder eine bei Laserdioden häufig schon integrierte Kollimatoroptik in das den Anschlußschlauch 3, das zahnärztliche Handstück 1 und eine Lichtsonde 2 umfassende Lichtleitersystem eingekoppelt. Mit Hilfe dieses Lichtleitersystems wird die Anregungsstrahlung 11 gezielt einem zu untersuchenden Bereich 19 des Zahns 8 zugeführt. Durch die Anregungsstrahlung wird in dem bestrahlten Zahnbereich 19 eine Fluoreszenzstrahlung 20 über einen relativen breiten Spektralbereich hervorgerufen, die von in der Lichtsonde 2 vorgesehenen Detektionsfasern erfaßt und über einen Strahlteiler 17, ein optisches Filter einer Fotodiode zur Erfassung der Fluoreszenzstrahlung zugeführt wird, deren Ausgangssignal wiederum einer Zentraleinheit 22 zugeführt ist. Das optische Filter und die Fotodiode sind in Fig. 2 nicht dargestellt, da sie auch in der Zentraleinheit 22 integriert sein können. Vorteilhafterweise weist das zahnärztliche Handstück 1 einen zentralen Kernlichtleiter zum Führen der Anregungsstrahlung 11 auf, der von mehreren koaxial angeordneten Detektionslichtleiterfasern zum Ableiten der erfaßten Fluoreszenzstrahlung umgeben ist, während die Lichtsonde 2 mehrere Emissionsfasern zum Bestrahlen des Zahns 8 mit der Anregungsstrahlung und mehrere Detektionsfasern zum Erfassen der von dem Zahn zurückgestrahlten Fluoreszenzstrahlung aufweist, wobei die Detektions- bzw. Emissionsfasern der Lichtsonde 2 mit den entsprechenden Detektionsfasern bzw. dem zentralen Kernlichtleiter verbunden sind. Der der von der Zentraleinheit 22 empfangen Fluoreszenzstrahlung entsprechende Momentanmeßwert wird in einer Anzeige 13 dargestellt. Aufgrund eines erhöhten Anzeigenwertes kann der Zahnarzt darauf schließen, daß es sich bei dem untersuchten Zahn 8 um einen kariösen Zahn handelt. Der exakte Bedienablauf der in Figur 2 gezeigten erfindungsgemäßen Vorrichtung zum Erkennen von Karies ist folgendermaßen.

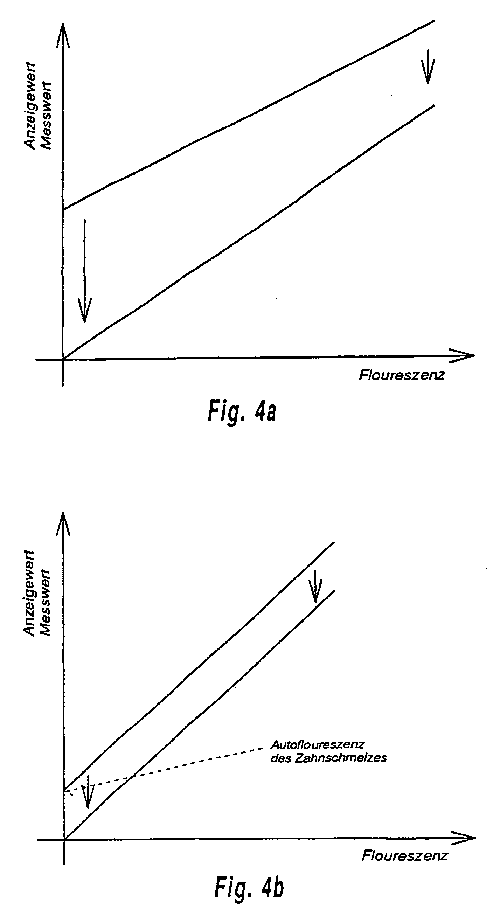

Zunächst wird eine bestimmte Lichtsonde 2 auf das zahnärztliche Handstück 2 aufgesetzt. Im vorliegenden Fall kann zwischen drei unterschiedlichen Lichtsonden, beispielsweise für die Untersuchung von glatten Zahnoberflächen, Zahnzwischenräumen oder Fissuren, gewählt werden. Wird eine Lichtsonde neu auf das zahnärztliche Handstück 1 aufgesetzt, kann die entsprechende Lichtsonde zunächst kalibriert werden. Zu diesem Zweck wird die ausgewählte Lichtsonde senkrecht mit ihrer Spitze auf zwei Referenzwerkstücke bzw. Kalibrierstandards mit unteschiedlichen Fluoreszenzwerten aufgesetzt. Die Referenzwerkstücke können beispielsweise aus Keramik gefertigt sein. Der Kalibriermodus wird durch Drücken der Taste 4 eingeleitet, wobei die Taste 4 so lange gedrückt gehalten werden muß bis die Zentraleinheit 22 ein Bestätigungssignal akustisch ausgibt. Nach erfolgreicher Kalibrierung bezüglich des ersten Referenzwerkstücks wird auf ähnliche Weise die Kalibrierung bezüglich eines zweiten Referenzwerkstücks durchgeführt. Der Vorgang des Kalibrierens ist beispielshaft in Figur 4a dargestellt. Daraus ist ersichtlich, daß mit Hilfe der Kalibrierung die ursprüngliche Meßkurve der gewählten Lichtsonde in eine direkt proportional mit der Fluoreszenz verlaufende Meßkurve transformiert wird, wobei die Verschiebung der ursprünglichen Meßkurve durch die Kalibrierung der beiden Referenzwerkstücke und den daraus gewonnenen Kalibrierstandardwerten abgeleitet wird. Die sich aus der Kalibrierung ergebenden Kalibrierstandardwerte werden in einem Speicher 23 gespeichert. Wird einer der beiden Kalibrierstandards bzw. Referenzwerkstücke ausgetauscht, muß der entsprechende Kalibrierstandardwert des neuen Referenzwerkstücks neu eingestellt werden. Zu diesem Zweck wird erneut die Taste 5 gedrückt und anschließend über den Ringschalter 9 der bisher eingestellte Kalibrierstandardwert verändert. Nach Veränderung des neuen Kalibrierstandardwertes wird dieser erneut im Speicher 23 gespeichert.

Der Typ der auf das zahnärztliche Handstück 1 aufgesteckten Lichtsonde muß vor dem eigentlichen Beginn der Messung zunächst der Vorrichtung mitgeteilt werden. Dies geschieht durch Betätigen der Taste 6, wobei abhängig von der Auswahl eine der drei Leuchtdioden 15a - 15c des Gehäuses 12 aufleuchtet.

Erfindungsgemäß wird jede Lichtsonde 2 vor deren Inbetriebnahme zunächst gegenüber der Eigenfluoreszenz des zu untersuchenden Zahnes abgeglichen. Da jeder Zahn eine unterschiedliche Eigenfluoreszenz aufweist, kann die Eigenfluoreszenz die Messung stören. Zum Abgleichen wird daher vor Beginn der eigentlichen Karieserkennung ein gesunder Zahn mit der ausgewählten Lichtsonde 2 vermessen. Der Abgleichvorgang wird durch langes Drücken (beispielsweise eine Sekunde) des Ringschalters 9 auf dem zahnärztlichen Handstück 1 eingeleitet. Mit dem Abgleich wird die Momentanmeßwertanzeige 13 auf Null gesetzt. Der Abgleichvorgang ist beispielshaft in Figur 4b dargestellt. Daraus ist ersichtlich, daß mit Hilfe des Abgleichvorganges der durch die Autofluoreszenz des zu untersuchenden Zahns hervorgerufene Offset beseitigt wird. Die Abgleichwerte für jede Lichtsonde 2 werden in einem Speicher 25 gespeichert, während nach Auswahl einer bestimmte Lichtsonde der entsprechende Lichtsondentyp und der entsprechenden Abgleichwert in einen Speicher 26 ausgelesen und anschließend für die eigentliche Messung verwendet werden.

Wie bereits erwähnt, wird während des eigentliche Meßbetriebs in der Anzeige 13 der jeweilige Momentanmeßwert der an dem untersuchten Zahn 8 angeregten Fluoreszenzstrahlung 20 angezeigt. Es ist jedoch auch eine akustische Anzeige des Meßwertes denkbar. Der Momentanwert wird jeweils mit einem bisher ermittelten Spitzenwert verglichen (Schaltungselement 29) und, falls der Momentanwert den bisherigen Spitzenwert übertrifft, als neuer Spitzenwert in einem Spitzenwertspeicher 31 gespeichert und in der Anzeige 14 dargestellt. Die Anzeige 14 zeigt somit stets den bisher ermittelten Spitzenwert der Kariesmessung an. Der Spitzenwert kann jederzeit durch einen kurzen Doppelklick, d.h. ein Doppelbetätigung innerhalb eines bestimmten Zeitintervalls, des Ringschalters 9 gelöscht werden. Nach Beendigung einer ersten Untersuchung des Zahns 8 kann somit festgestellt werden, ob der untersuchte Zahn kariös ist und der der Zahnstelle mit dem stärksten Kariesbefall entsprechende Meßwert wird als Spitzenwert in der Anzeige 14 angezeigt.

Nach Beendigung der Untersuchung des Zahns 8 muß zur eigentlichen Behandlung der kariösen Stelle des Zahns 8 die Zahnstelle mit dem stärksten Kariesbefall wieder aufgefunden werden können. Erfindungsgemäß wird dies dadurch erreicht, daß der Zahn 8 erneut mit der Anregungsstrahlung 11 abgetastet und der in der Anzeige 13 dargestellte Momentanmeßwert mit dem Spitzenwert der Anzeige 14 verglichen wird. Der Vergleich kann dabei visuell oder automatisch erfolgen. Entspricht der in der Anzeige 13 angezeigte Momentanmeßwert dem in der Anzeige 14 angezeigten Spitzenwert, so wurde die Zahnstelle mit dem stärksten Kariesbefall wieder aufgefunden. Um das Auffinden der am stärksten mit Karies befallenen Zahnstelle zu erleichtern, wird erfindungsgemäß vorgeschlagen, bei dem erneuten Abtasten des Zahns 8 ein akustisches Signal über einen Lautsprecher 30 auszugeben, dessen Frequenz sich mit veränderndem Meßwert erhöht oder senkt. Alternativ kann vorgesehen sein, daß dieses akustische Signal nur ausgegeben wird, solange der Meßwert ansteigt. Auf diese Weise läßt sich die Stelle mit dem stärksten Kariesbefall besonders schnell auffinden. Ebenso kann vorgesehen sein, daß das akustische Signal erst dann ausgegeben wird, wenn der in der Anzeige 13 angezeigte Meßwert dem in der Anzeige 14 angezeigten Spitzenwert entspricht. Über die in Figur 1 gezeigte Taste 7 des Gehäuses 12 können die unterschiedlichen akustischen Signalmodi ausgewählt werden. Insbesondere kann auch über diese Taste 7 die Ausgabe eines akustischen Signals abgeschaltet werden.

Wie bereits erwähnt, wird vorteilhafterweise die erfindungsgemäße Vorrichtung zum Erkennen von Karies mit einer Batterie oder einem Akkumulator 21 als Spannungsquelle betrieben. Während des Betriebs wird die von der Spannungsquelle 21 abgegebene Versorgungsspannung regelmäßig durch eine entsprechende Überwachungseinrichtung 27 überprüft und, falls die Versorgungsspannung unter einen vorgegebenen Grenzwert abgesunken ist, die in dem Gehäuse 12 vorgesehene Leuchtdiode 16 eingeschaltet. Um die Spannungsquelle 21 zu schonen, verfügt die erfindungsgemäße Vorrichtung über eine automatische Selbstabschaltung 33, die die Spannungsversorgung unterbricht, wenn während eines vorgegebenen Zeitintervalls keine Messung durchgeführt oder keine Taste betätigt worden ist.

Des weiteren kann eine Einrichtung 34 zur Erfassung der Gesamtbetriebszeit der erfindungsgemäßen Vorrichtung vorgesehen sein.

Um einen gealterten oder defekten Laser 10 erkennen zu können, ist zudem eine Einrichtung 35 vorgesehen, die kontinuierlich den Laserstrom des Lasers 10 überwacht und über einen Lautsprecher 36 ein entsprechendes Warnsignal ausgibt, falls der erfaßt Laserstrom unter einen vorgegebenen Grenzwert liegt oder einen vorgegebenen Grenzwert übersteigt.

Es sei darauf hingewiesen, daß die Speicher 23, 25 und 26 als nicht-flüchtige Speicher ausgelegt sind, so daß die darin gespeicherten Kalibrierstandardwerte, Abgleichwerte sowie der Typ der zuletzt verwendeten Lichtsonde auch nach Ausschalten der Vorrichtung erhalten bleiben. Somit kann nach erneutem Starten der erfindungsgemäßen Vorrichtung mit denselben Einstellung wie vor dem Abschalten der Vorrichtung weitergearbeitet werden.

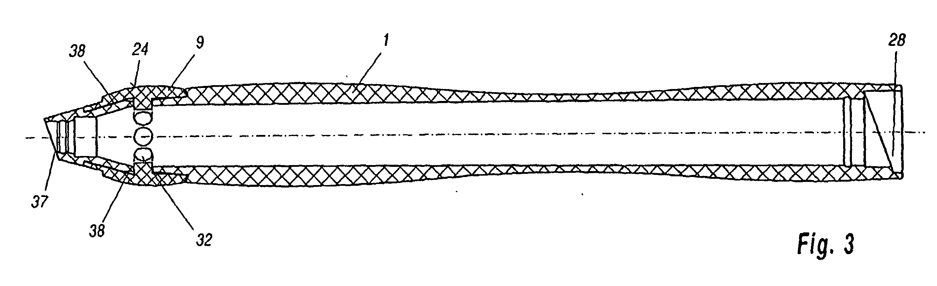

Fig. 3 zeigt abschließend eine Querschnittsansicht des in Fig. 1 gezeigten zahnärztlichen Handstücks, wobei insbesondere der Ringschalter 9 im Detail dargestellt ist. Dieser Ringschalter 9 umfaßt eine sich ringförmig um das zahnärztliche Handstück 1 erstreckende Betätigungsfläche 24, die innen gegenüber Kontaktfedern 38 abgestützt ist. Durch Drücken der Betätigungsfläche 24 werden Kontaktmittel 32 aktiviert, die abhängig vom eingestellten Betriebsmodus die oben beschriebenen Funktionen ausführen. Die gedrückte Betätigungsfläche 24 wird nach deren Loslassen von den Kontaktfedern 38 wieder in die Ausgangsposition nach außen gedrückt. Der in Fig. 3 gezeigte Ringschalter 9 weist den Vorteil auf, daß durch einen einzigen Fingerdruck der Schalter unabhängig von der Lage des Handstücks 1 in der Hand des Zahnarztes einfach betätigt werden kann. An seinen beiden Längsenden besitzt das zahnärztliche Handstück 1 Hohlräume 37 bzw. 28 zur Aufnahme der Lichtsonde bzw. des Versorgungsschlauches.

Um die von der erfindungsgemäßen Vorrichtung erfaßten Fluoreszenzmeßwerte auch rechnergestützt verarbeiten zu können, kann eine geeignete Schnittstelle 39 für eine Datenübertragung an einen externen Rechner (Personal Computer) vorgesehen sein. Insbesondere kann zur Datenübertragung ein Optokoppler anstelle einer elektrischen Datenübertragungsstrecke verwendet werden.

## Patentansprüche

1. Verfahren zum Lokalisieren einer Stelle mit erhöhter Fluoreszenzaktivität an einem zu untersuchenden Objekt (8) in Form eines menschlichen oder tierischen körpergewebes, insbesondere an einem Zahn, umfassend die Schritte:
a) Bestrahlen des zu untersuchenden Objekts (8) mit einer Anregungsstrahlung (11),
b) Erfassen eines Momentanmeßwertes der durch die Anregungsstrahlung an dem Objekt hervorgerufenen Fluoreszenzstrahlung (20),
c) Vergleichen des Momentanmeßwertes mit einem während des Meßbetriebs zuvor ermittelten Spitzenmeßwert der Fluoreszenzstrahlung, der einem Bereich des untersuchten Objekts mit der stärksten Fluoreszenzaktivität entspricht, und
d) Lokalisieren des Bereichs mit der stärksten Fluoreszenzaktivität, wenn der Momentanmeßwert mit dem Spitzenmeßwert übereinstimmt.

2. Verfahren nach Anspruch 1,
**dadurch gekennzeichnet,**
**daß** der Spitzenmeßwert ermittelt wird durch:
- Erfassen eines Momentanmeßwertes der durch die Anregungsstrahlung (11) an dem untersuchten Objekt (8) hervorgerufenen Fluoreszenzstrahlung (20),
- Vergleichen des Momentanmeßwertes mit einem bisherigen Spitzenmeßwert und Speichern des Momentanmeßwertes als neuer Spitzenmeßwert, falls der Momentanmeßwert größer als der bisherige Spitzenmeßwert ist, und
- Wiederholen dieser Schritte bis zum Ende der Messung.

3. Verfahren nach Anspruch 1 oder 2,
**dadurch gekennzeichnet,**
**daß** vor Beginn der Messung ein Abgleich durchgeführt wird, wodurch die Messung an die Eigenfluoreszenz des zu untersuchenden Objekts (8) angepaßt und der Momentanmeßwert auf Null gesetzt wird.

4. Verfahren nach einem der Ansprüche 1 - 3,
**dadurch gekennzeichnet,**
**daß** die Fluoreszenzstrahlung von entsprechenden Erfassungsmitteln (2) erfaßt wird, wobei vor Verwendung von neuen Erfassungsmitteln eine Kalibrierung der neuen Erfassungsmittel anhand zweier vordefinierter Referenzwerkstücke durchgeführt wird.

5. Verfahren nach Anspruch 3 und 4,
**dadurch gekennzeichnet,**
**daß** die Kalibrierung in einem Kalibrierbetrieb über ein Schaltelement (9) durchgeführt wird, daß der Abgleich in einem Meßbetrieb durch dasselbe Schalteelement (9) durchgeführt wird, indem das Schaltelement für eine bestimmte Zeitspanne betätigt wird, und daß in dem Meßbetrieb ein gespeicherter Spitzenmeßwert gelöscht wird, indem dasselbe Schaltelement (9) innerhalb eines bestimmten Zeitintervalls mehrfach betätigt wird.

6. Vorrichtung zum Ermitteln von Karies, Plaque oder bakteriellen Befall an Zähnen, die mit Hilfe einer Versorgungsspannung betrieben wird, mit
• Mitteln (10) zum Erzeugen einer Anregungsstrahlung (11), mit der ein zu untersuchender Zahn (8) zu bestrahlen ist,
• Erfassungsmitteln (2) zum Erfassen eines Momentanmeßwerts der durch die Anregungsstrahlung an dem Zahn hervorgerufenen Fluoreszenzmessung (20) und
• Speichermitteln (31) zum Speichern des über eine bestimmte Meßperiode erzielten Spitzenmeßwertes der erfaßten Fluoreszenzstrahlung (20),
**gekennzeichnet durch**
Mittel (29) zum Vergleich des Momentanmeßwertes mit dem Spitzenmeßwert.

7. Vorrichtung nach Anspruch 6,
**gekennzeichnet durch**
Anzeigemittel (13, 14) zum Anzeigen des Momentanmeßwerts und des über die bestimmte Meßperiode erzielten Spitzenmeßwerts der Fluoreszenzstrahlung.

8. Vorrichtung nach Anspruch 6 oder 7,
**gekennzeichnet durch**
Mittel (9) zum Löschen der den Spitzenmeßwert speichernden Speichermittel (31).

9. Vorrichtung nach einem der Ansprüche 6 - 8,
**gekennzeichnet durch**
Mittel (9) zum Abgleichen der Vorrichtung, wodurch die Vorrichtung an die Eigenfluoreszenz des zu untersuchenden Zahnes (8) angepaßt wird.

10. Vorrichtung nach Anspruch 8 oder 9,
**dadurch gekennzeichnet,**
**daß** die Mittel zum Löschen der den Spitzenmeßwert speichernden Speichermittel (31) und die Mittel zum Abgleichen der Vorrichtung durch ein und dasselbe Schaltelement (9) realisiert sind, dessen Funktion abhängig von seiner Betätigungsdauer und Betätigungsfolge festgelegt ist.

11. Vorrichtung nach Anspruch 10,
**dadurch gekennzeichnet,**
**daß** das Schaltelement (9) durch einen sich mit seiner Schaltfläche ringförmig um ein zahnärztliches Handstück (1) erstreckenden Schalter gebildet ist, wobei das zahnärztliche Handstück (1) zum Richten der Anregungsstrahlung (11) dient und die Erfassungsmittel (2) trägt.

12. Vorrichtung nach einem der Ansprüche 6 - 11,
**gekennzeichnet durch**
Mittel (1) zum Kalibrieren der Erfassungsmittel (2) anhand eines Referenzwerkstücks oder zweier Referenzwerkstücke, welche insbesondere aus Keramik gefertigt sind.

13. Vorrichtung nach einem der Ansprüche 6 - 12,
**gekennzeichnet durch**
eine oben und/oder seitlich an ein Gehäuse (12) der Vorrichtung anbringbare Halterung (19) für ein zahnärztliches Handstück (1) der Vorrichtung, wobei die Halterung in Stufen oder stufenlos drehbar ist.

14. Vorrichtung nach einem der Ansprüche 6 - 13,
**dadurch gekennzeichnet,**
**daß** die Erfassungsmittel (2) austauschbar sind, und daß Anzeigemittel (15a - 15c) zur Anzeige der jeweils gewählten Erfassungsmittel vorhanden sind.

15. Vorrichtung nach Anspruch 14,
**gekennzeichnet durch**
Mittel (6) zur Auswahl der Erfassungsmittel (2).

16. Vorrichtung nach Anspruch 14 oder 15,
**gekennzeichnet durch**
Speichermittel (26) zum Speichern des zuletzt verwendeten Erfassungsmittels (2).

17. Vorrichtung nach einem der Ansprüche 14 - 16 und Anspruch 9,
**gekennzeichnet durch**
Speichermittel (25) zum Speichern der Abgleichwerte der einzelnen Erfassungsmittel (2).

18. Vorrichtung nach einem der Ansprüche 14 - 17 und Anspruch 12,
**gekennzeichnet durch**
Speichermittel (23) zum Speichern der Kalibrierwerte der einzelnen Erfassungsmittel (2).

19. Vorrichtung nach einem der Ansprüche 6 - 18,
**gekennzeichnet durch**
Mittel (16) zum Abgeben eines Warnsignals, falls die Versorgungsspannung der Vorrichtung unter einen bestimmten Grenzwert abgesunken ist.

20. Vorrichtung nach einem der Ansprüche 6 - 19,
**gekennzeichnet durch**
Mittel (33) zum automatischen Abschalten einer die Versorgungsspannung bereitstellenden Spannungsversorgung (21) der Vorrichtung, falls die Vorrichtung nicht innerhalb eines bestimmten Zeitintervalls betätigt worden ist.

21. Vorrichtung nach einem der Ansprüche 6 - 20,
**gekennzeichnet durch**
Mittel (34) zum Erfassen der Gesamtbetriebszeit der Vorrichtung.

22. Vorrichtung nach einem der Ansprüche 6 - 21,
**dadurch gekennzeichnet,**
**daß** die Mittel (10) zum Erzeugen der Anregungsstrahlung (11) Laserlicht als die Anregungsstrahlung erzeugen, und daß Mittel (35) zum Überwachendes Laserstroms der das Laserlicht erzeugenden Mittel (10) vorhanden sind, die ein Warnsignal erzeugen, falls der Laserstrom unter einen bestimmten Grenzwert abgesunken oder über einen bestimmten Grenzwert angestiegen ist.

23. Vorrichtung nach einem der Ansprüche 6 - 22,
**gekennzeichnet durch**
Mittel (30) zur Ausgabe eines von dem Momentanmeßwert abhängigen akustischen Signals, wobei die Frequenz des Signals mit dem Momentanmeßwert ansteigt.

24. Vorrichtung nach Anspruch 23,
**dadurch gekennzeichnet,**
**daß** das akustische Signal nur ausgegeben wird, falls der Momentanmeßwert ansteigt.

25. Vorrichtung nach Anspruch 23,
**dadurch gekennzeichnet,**
**daß** das akustische Signal nur ausgegeben wird, falls der Momentanmeßwert mit dem Spitzenmeßwert übereinstimmt.

26. Vorrichtung nach einem der Ansprüche 6 - 25,
**dadurch gekennzeichnet,**
**daß** Mittel (39) zum Übertragender von den Erfassungsmitteln (2) erfaßten Meßwerte an einen anschließbaren Rechner vorhanden sind.

27. Vorrichtung nach Anspruch 26,
**dadurch gekennzeichnet,**
**daß** die Übertragungsmittel (39) einen Optokoppler umfassen.

28. Vorrichtung nach einem der Ansprüche 6 bis 27,
**gekennzeichnet durch**
eine Spannungsversorgungseinrichtung (21), die die Versorgungsspannung für den Betrieb der Vorrichtung liefert und **durch** einen Akkumulator oder eine Batterie gebildet ist.

29. Vorrichtung nach einem der Ansprüche 6 bis 28,
**gekennzeichnet durch**
Befestigungsmittel zur Befestigung der Vorrichtung an einem zahnärztlichen Arbeitsplatz.

30. Vorrichtung nach Anspruch 29,
**dadurch gekennzeichnet,**
**daß** die Befestigungsmittel derart ausgestaltet sind, daß ein Verdrehen und/oder Kippen der Vorrichtung an dem Arbeitsplatz möglich ist.

## Claims

1. Method for localising on an object to be investigated, in the form of human or animal body tissue, in particular on a tooth, a site having increased fluorescence activity. including the steps:-
a) irradiating the object (8) to be investigated with an excitation radiation (11),
b) detecting an instantaneous measurement value of the fluorescence radiation (20) brought about at the object by the excitation radiation,
c) comparing the instantaneous measurement value with a peak measurement value of the fluorescence radiation previously detected during the measurement operation, which corresponds to a region of the investigated object with the strongest fluorescence activity, and
d) localising the region with the strongest fluorescence activity, when the instantaneous measurement value coincides with the peak measurement value.

2. Method according to claim 1,
**characterised in that**,
the peak measurement value is determined by:
- detecting an instantaneous measurement value of the fluorescence radiation (20) brought about at the investigated object (8) by the excitation radiation (11),
- comparing the instantaneous measurement value with the previous peak measurement value and storing the instantaneous measurement value as new peak measurement value if the instantaneous measurement value is greater than the previous peak measurement value, and
- repeating these steps until the end of the measurement.

3. Method according to claim 1 or 2,
**characterised in that**,
before the beginning of the measurement a compensation is effected by which the measurement is adapted to the self-fluorescence of the object (8) to be investigated and the instantaneous measurement value is set to zero.

4. Method according to any of claims 1 to 3,
**characterised in that**,
the fluorescence radiation is acquired by appropriate acquisition means (2), whereby before employment of new acquisition means a calibration of the new acquisition means is effected on the basis of two predefined reference objects.

5. Method according to claim 3 and 4,
**characterised in that**,
the calibration is effected in a calibration operation via a switching element (9),
**in that** the compensation is effected in a measurement operation by means of the same switching element (9), by the switching element being actuated for a particular period of time, and
**in that** in the measurement operation a stored peak measurement value is deleted when the same switching element (9) is actuated several times within a predetermined interval of time.

6. Device for the determination of caries, plaque or bacterial infection of teeth, which is operated with the aid of a supply voltage, having
• means (10) for generating an excitation radiation (11), with which a tooth (8) to be investigated is to be irradiated,
• acquisition means (2) for acquiring an instantaneous measurement value of the fluorescence radiation (20) brought about at the tooth by the excitation radiation, and
• memory means (31) for storing the peak measurement value of the acquired fluorescence radiation (20) attained over a particular measurement period,
**characterised by**
means (29) for comparing the instantaneous measurement value with the peak measurement value.

7. Device according to claim 6,
**characterised by**
display means (13, 14) for displaying the instantaneous measurement value and the peak measurement value of the fluorescence radiation attained over the particular measurement period.

8. Device according to claim 6 or 7,
**characterised by**
means (9) for erasing the memory means (31) storing the peak measurement value.

9. Device according to any of claims 6 to 8,
**characterised by**,
means (9) for compensation of the device, whereby the device is adapted to the self-fluorescence of the tooth (8) to be investigated.

10. Device according to claim 8 or 9,
**characterised in that**,
the means for erasing the memory means (31) storing the peak measurement value and the means for compensation of the device are realised through one and the same switching element (9), the function of which is determined in dependence upon its duration of actuation and sequence of actuation.

11. Device according to claim 10,
**characterised in that**,
the switching element (9) is constituted by a switch which extends with its switch surface ring-like around a dental handpiece (1), the dental handpiece (1) serving for directing the excitation radiation (11) and carrying the acquisition means (2).

12. Device according to any of claims 6 to 11,
**characterised by**
means (1) for calibrating the acquisition means (2) on the basis of a reference object or two reference objects which are in particular of ceramics.

13. Device according to any of claims 6 to 12,
**characterised by**
a holder (19) for a dental handpiece (1) of the device, which holder can be applied to a housing (12) of the device above or to the side, the holder being rotatable in steps or steplessly.

14. Device according to any of claims 6 to 13,
**characterised in that**,
the acquisition means (2) are exchangeable, and
**in that** display means (15a-15c) are present for the display of the respective selected acquisition means.

15. Device according to claim 14,
**characterised by**
means (6) for selecting the acquisition means (2).

16. Device according to claim 14 or 15,
**characterised by**
memory means (26) for storing the last employed acquisition means (2).

17. Device according to any of claims 14 to 16 and claim 9,
**characterised by**
memory means (25) for storing the compensation values of the individual acquisition means (2).

18. Device according to any of claims 14 to 17 and claim 12,
**characterised by**
memory means (23) for storing the calibration values of the individual acquisition means (2).

19. Device according to any of claims 6 to 18,
**characterised by**
means (16) for emitting a warning signal if the supply voltage of the device has fallen below a particular limit value.

20. Device according to any of claims 6 to 19,
**characterised by**
means (33) for automatic switching off of a voltage supply (21) of the device, making available the supply voltage, if the device is not actuated within a particular time interval.

21. Device according to any of claims 6 to 20,
**characterised by**
means (34) for determining the total operating time of the device.

22. Device according to any of claims 6 to 21,
**characterised in that**,
the means (10) for generating the excitation radiation (11) generate laser light as the excitation radiation, and
**in that** means (35) for monitoring the laser current of the means (10) generating the laser light are present which generate a warning signal if the laser current has fallen below a particular limit value or has increased above a particular limit value.

23. Device according to any of claims 6 to 22,
**characterised by**
means (30) for emitting an acoustic signal dependent upon the instantaneous measurement value, the frequency of the signal increasing with the instantaneous measurement value.

24. Device according to claim 23,
**characterised in that**,
the acoustic signal is emitted only if the instantaneous measurement value increases.

25. Device according to claim 23,
**characterised in that**,
the acoustic signal is emitted only if the instantaneous measurement value coincides with the peak measurement value.

26. Device according to any of claims 6 to 25,
**characterised in that**,
there are present means (39) for the transmission of the measurement values acquired by the acquisition means (2) to a connectable computer.

27. Device according to claim 26,
**characterised in that**,
the transmission means (39) include an optical coupler.

28. Device according to any of claims 6 to 27,
**characterised by**
a voltage supply device (21) which delivers the supply voltage for the operation of the device and is constituted by an accumulator or a battery.

29. Device according to any of claims 6 to 28,
**characterised by**
attachment means for attaching the device at a dental work station.

30. Device according to claim 29,
**characterised in that**,
the attachment means are so configured that a rotation or tilting of the device at the work station is possible.

## Revendications

1. Procédé de localisation d'une zone ayant une activité de fluorescence accrue sur un objet à examiner (8) ayant la forme d'un tissu corporel humain ou animal, en particulier sur une dent, comportant les étapes :
a) irradiation de l'objet à examiner (8) avec un rayon d'excitation (11),
b) enregistrement d'une valeur instantanée du rayonnement de fluorescence (20) provoqué sur l'objet par le rayon d'excitation,
c) comparaison de la valeur instantanée avec une valeur de pointe du rayonnement de fluorescence déterminée au préalable pendant la mesure, qui correspond à une région de l'objet examiné ayant la plus forte activité de fluorescence et
d) localisation de la région ayant la plus forte activité de fluorescence lorsque la valeur instantanée coïncide avec la valeur de pointe.

2. Procédé selon la revendication 1,
**caractérisé en ce que,**
la valeur de pointe est déterminée par :
- enregistrement d'une valeur instantanée du rayonnement de fluorescence (20) provoqué sur l'objet examiné par le rayon d'excitation,
- comparaison de la valeur instantanée avec la valeur de pointe antérieurement trouvée et mémorisation de la valeur instantanée en tant que nouvelle valeur de pointe si la valeur instantanée est supérieure à la valeur de pointe antérieurement trouvée et
- répétition de ces étapes jusqu'à la fin de l'opération de mesure.

3. Procédé selon la revendication 1 ou 2,
**caractérisé en ce que**,
on procède à un ajustement avant de commencer la mesure, moyennant quoi la mesure est adaptée à la fluorescence propre de l'objet à examiner (8) et la valeur instantanée remise à zéro.

4. Procédé selon l'une quelconque des revendications 1 à 3,
**caractérisé en ce que,**
le rayonnement de fluorescence des moyens de détection correspondants (2) est détecté, un calibrage des nouveaux moyens de détection étant réalisé à l'aide de deux pièces de référence prédéfinies avant l'utilisation de nouveaux moyens de détection.

5. Procédé selon les revendications 3 et 4,
**caractérisé en ce que**,
le calibrage est réalisé en mode de calibrage via un élément de commutation (9), **en ce que** l'alignement est réalisé en mode de mesure par le même élément de commutation (9) en activant l'élément de commutation pendant un temps donné, et **en ce qu'**en mode de mesure une valeur de pointe mémorisée est supprimée en actionnant plusieurs fois le même élément de commutation (9) dans les limites d'un intervalle de temps donné.

6. Dispositif pour déterminer la présence de caries, de plaque dentaire ou d'attaque bactérienne sur des dents qui fonctionne à l'aide d'une tension d'alimentation, ayant
• des moyens (10) pour produire un rayon d'excitation (11) servant à irradier une dent à examiner (8),
• des moyens de détection (2) pour la détection d'une valeur instantanée du rayonnement de fluorescence (20) provoqué sur l'objet par le rayon d'excitation et
• des moyens de mémorisation (31) pour mémoriser la valeur de pointe du rayonnement de fluorescence détecté (20) obtenue pendant une période de mesure donnée,
**caractérisé par**
des moyens (29) permettant la comparaison de la valeur instantanée avec la valeur de pointe.

7. Dispositif selon la revendication 6,
**caractérisé par**
des moyens d'affichage (13, 14) permettant l'affichage de la valeur instantanée et de la valeur de pointe du rayonnement de fluorescence obtenue pendant la période de mesure donnée.

8. Dispositif selon la revendication 6 ou 7,
**caractérisé par**
des moyens (9) permettant l'effacement des moyens de mémorisation (31) qui mémorisent la valeur de pointe.

9. Dispositif selon l'une quelconque des revendications 6 à 8,
**caractérisé par**
des moyens (9) permettant l'ajustement du dispositif, moyennant quoi le dispositif est adapté à la fluorescence propre de la dent à examiner (8).

10. Dispositif selon la revendication 8 ou 9,
**caractérisé en ce que,**
les moyens permettant l'effacement des moyens de mémorisation (31) mémorisant la valeur de pointe et les moyens permettant l'ajustement du dispositif sont réalisés par un et même élément de commutation (9) dont la fonction est déterminée en fonction de sa durée d'activation et de sa séquence d'activation.

11. Dispositif selon la revendication 10,
**caractérisé en ce que,**
l'élément de commutation (9) est formé par un commutateur s'étendant en forme d'anneau, avec sa face de commutation, autour d'un porte-outil dentaire (1), le porte-outil dentaire (1) servant à redresser le rayon d'excitation (11) et portant les moyens de détection (2).

12. Dispositif selon l'une quelconque des revendications 6 à 11,
**caractérisé par,**
des moyens (1) permettant le calibrage des moyens de détection (2) à l'aide d'une pièce de référence ou de deux pièces de référence qui sont fabriquées en particulier en céramique.

13. Dispositif selon l'une quelconque des revendications 6 à 12,
**caractérisé par,**
une fixation (19) destinée à un porte-outil dentaire (1) du dispositif, pouvant être disposée en haut et/ou sur le côté d'un boîtier (12) du dispositif, la fixation pouvant tourner par paliers ou en continu.

14. Dispositif selon l'une quelconque des revendications 6 à 13,
**caractérisé en ce que**,
les moyens de détection (2) sont interchangeables et **en ce que** des moyens d'affichage (15a - 15c) permettant l'affichage des moyens de détection choisis sont présents.

15. Dispositif selon la revendication 14,
**caractérisé par**
des moyens (6) permettant de choisir les moyens de détection (2).

16. Dispositif selon la revendication 14 ou 15,
**caractérisé par**
des moyens de mémorisation (26) permettant la mémorisation du dernier moyen de détection (2) utilisé.

17. Dispositif selon l'une quelconque des revendications 14 à 16 et la revendication 9,
**caractérisé par**,
des moyens de mémorisation (25) permettant la mémorisation des valeurs d'ajustement des différents moyens de détection (2).

18. Dispositif selon l'une quelconque des revendications 14 à 17 et la revendication 12,
**caractérisé par**,
des moyens de mémorisation (23) permettant la mémorisation des valeurs de calibrage des différents moyens de détection (2).

19. Dispositif selon l'une quelconque des revendications 6 à 18,
**caractérisé par**,
des moyens (16) permettant l'émission d'un signal d'alerte si la tension d'alimentation du dispositif est tombée en dessous d'une valeur limite donnée.

20. Dispositif selon l'une quelconque des revendications 6 à 19,
**caractérisé par**,
des moyens (33) permettant la déconnexion automatique d'une alimentation en tension (21) du dispositif fournissant la tension d'alimentation, si ce dispositif n'est pas activé dans un intervalle de temps donné.

21. Dispositif selon l'une quelconque des revendications 6 à 20,
**caractérisé par**,
des moyens (34) permettant la détection du temps de fonctionnement total du dispositif.

22. Dispositif selon l'une quelconque des revendications 6 à 21,
**caractérisé en ce que**,
les moyens (10) permettant la production d'un rayon d'excitation (11) produisent de la lumière laser en tant que rayon d'excitation et **en ce que** des moyens (35) destinés au courant laser de surveillance des moyens (10) produisant la lumière laser sont présents, qui produisent un signal d'alerte si le courant laser est baissé en dessous d'une valeur limite donnée ou est augmenté en dessus d'une valeur limite donnée.

23. Dispositif selon l'une quelconque des revendications 6 à 22,
**caractérisé par**,
des moyens (30) permettant l'émission d'un signal acoustique dépendant de la valeur instantanée, la fréquence du signal augmentant avec la valeur instantanée.

24. Dispositif selon la revendication 23,
**caractérisé en ce que**,
le signal acoustique n'est émis que si la valeur instantanée augmente.

25. Dispositif selon la revendication 23,
**caractérisé en ce que**,
le signal acoustique n'est émis que si la valeur instantanée coïncide avec la valeur de pointe.

26. Dispositif selon l'une quelconque des revendications 6 à 25,
**caractérisé en ce que**,
des moyens (39) permettant la transmission à un calculateur des valeurs détectées par les moyens de détection (2) pouvant être raccordé, sont présents.

27. Dispositif selon la revendication 26,
**caractérisé en ce que**,
les moyens de transmission (39) comportent un optocoupleur.

28. Dispositif selon l'une quelconque des revendications 6 à 27,
**caractérisé par**,
un dispositif d'alimentation en tension (21) qui fournit la tension d'alimentation pour le fonctionnement du dispositif et est constitué d'un accumulateur ou d'une batterie.

29. Dispositif selon l'une quelconque des revendications 6 à 28
**caractérisé par,**
des moyens de fixation permettant la fixation du dispositif sur un poste de travail dentaire.

30. Dispositif selon la revendication 29,
**caractérisé en ce que**,
les moyens de fixation sont équipés de manière à permettre la rotation et/ou le basculement du dispositif sur le poste de travail.
